# EUROPÄISCHE PATENTANMELDUNG

(11) **EP 1 930 032 A2**
(43) Veröffentlichungstag der Anmeldung: **11.06.2008**
(21) Anmeldenummer: 07015664.1
(22) Anmeldetag: 09.08.2007
(51) Int. Cl.: A61L 27/04, A61L 27/34, A61L 31/02, A61L 31/10

(54) **Biokorrodierbares metallisches Implantat mit einer Beschichtung oder Kavitätenfüllung aus Gelatine**

(30) Priorität: 06.09.2006 DE 102006042313
(71) Anmelder: BIOTRONIK VI Patent AG, 6341 Baar (CH)
(72) Erfinder: Bertsch, Torben, 90419 Nürnberg (DE); Borck, Alexander, Dr., 91086 Aurachtal (DE)
(74) Vertreter: Lindner-Vogt, Karin L.

(57) **Zusammenfassung**

Die Erfindung betrifft ein Implantat aus einem biokorrodierbaren metallischen Werkstoff und mit einer Beschichtung oder Kavitätenfüllung bestehend aus oder enthaltend Gelatine.

## Beschreibung

Die Erfindung betrifft (i) ein Implantat aus einem biokorrodierbaren metallischen Werkstoff, das eine Beschichtung oder Kavitätenfüllung aufweist, die aus Gelatine besteht oder dieses enthält, sowie (ii) eine neue Verwendung von Gelatine.

Implantate haben in vielfältiger Ausführungsform Anwendung in der modernen Medizintechnik gefunden. Sie dienen beispielsweise der Unterstützung von Gefäßen, Hohlorganen und Gangsystemen (Endovaskuläre Implantate), zur Befestigung und temporären Fixierung von Gewebeimplantaten und Gewebstransplantationen, aber auch zu orthopädischen Zwecken, zum Beispiel als Nagel, Platte oder Schraube.

So hat sich zum Beispiel die Implantation von Stents als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die tragende Umfangswandung wird in der Regel von einer gitterartigen Tragstruktur gebildet, die es erlaubt, den Stent in einem komprimierten Zustand mit kleinem Außendurchmesser bis zur behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Um unnötige Gefäßbeschädigungen zu vermeiden, sollte der Stent nach dem Aufweiten und nach Entfernen des Ballons nicht oder nur in geringem Umfang elastisch zurückfedern, so dass der Stent beim Aufweiten nur wenig über den gewünschten Enddurchmesser hinaus geweitet werden muss. Weitere Kriterien, die in Bezug auf einen Stent wünschenswert sind, umfassen beispielsweise eine gleichmäßige Flächenabdeckung und eine Struktur, die eine gewisse Flexibilität in Bezug auf die Längsachse des Stents erlaubt. In der Praxis wird zur Realisation der genannten mechanischen Eigenschaften der Stent in der Regel aus einem metallischen Werkstoff geformt.

Neben den mechanischen Eigenschaften eines Stents sollte dieser aus einem biokompatiblen Material bestehen, um Abstoßungsreaktionen vorzubeugen. Derzeit werden bei etwa 70 % aller perkutanen Interventionen Stents eingesetzt, in 25 % aller Fälle kommt es jedoch zu einer In-Stent Restenose aufgrund eines überschießenden neointimalen Wachstums, das durch eine starke Proliferation der arteriellen glatten Muskelzellen und eine chronische Entzündungsreaktion hervorgerufen wird. Zur Senkung der Restenoseraten werden verschiedene Lösungsansätze verfolgt.

Ein Ansatz zur Minderung der Restenoserate sieht vor, auf dem Stent eine pharmazeutisch aktive Substanz (Wirkstoff) bereitzustellen, die den Mechanismen der Restenose entgegenwirkt und den Heilungsverlauf unterstützt. Der Wirkstoff wird in Reinform oder eingebettet in eine Trägermatrix als Beschichtung aufgetragen oder in Kavitäten des Implantats gefüllt. Beispiele umfassen die Wirkstoffe Sirolimus und Paclitaxel.

Ein weiterer, aussichtsreicher Ansatz zur Lösung des Problems liegt in der Verwendung biokorrodierbarer Metalle und deren Legierungen, denn zumeist ist eine dauerhafte Stützfunktion durch den Stent nicht erforderlich; das zunächst geschädigte Körpergewebe regeneriert. So wird beispielsweise in DE 197 31 021 A1 vorgeschlagen, medizinische Implantate aus einem metallischen Werkstoff zu formen, dessen Hauptbestandteil Eisen, Zink oder Aluminium sind bzw. ein Element aus der Gruppe der Alkalimetalle oder Erdalkalimetalle. Als besonders geeignet werden Legierungen auf Basis von Magnesium, Eisen und Zink beschrieben. Nebenbestandteile der Legierungen können Mangan, Kobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90 %, Yttrium 3,7 - 5,5 %, Seltenerdmetallen 1,5 - 4,4 % und Rest < 1 % bekannt, die sich insbesondere zur Herstellung einer Endoprothese, z. B. in Form eines selbstexpandierenden oder ballonexpandierbaren Stents, eignet. Der Einsatz von biokorrodierbaren metallischen Werkstoffen in Implantaten dürfte zu einer deutlichen Minderung von Abstoßungs- oder Entzündungsreaktionen führen.

Die Kombination aus Wirkstofffreisetzung und biokorrodierbarem metallischen Wirkstoff erscheint besonders aussichtsreich. Der Wirkstoff wird als Beschichtung aufgebracht oder in eine Kavität im Implantat eingebracht - zumeist eingebettet in eine Trägermatrix. Bekannt sind beispielsweise Stents aus einer biokorrodierbaren Magnesiumlegierung mit einer Beschichtung aus einem Poly(L-lactid). Ungeachtet der erreichten Fortschritte sind jedoch noch folgende Probleme zu lösen:

Die Abbauprodukte der Trägermatrix sollen keinen merklichen Einfluss auf den lokalen pH-Wert besitzen, um einerseits ungewünschte Gewebsreaktionen zu vermeiden und andererseits den Einfluss auf den Korrosionsprozess des metallischen Implantatswerkstoffs zu mindern. Weiterhin sollte die Degradation der Trägermatrix schneller erfolgen als die Degradation des Grundkörpers, um unerwünschten Interaktionen der beiden Prozesse vorzubeugen.

Der Erfindung liegt die Aufgabe zugrunde, die geschilderten Nachteile des Standes der Technik zu überwinden.

Ein erster Aspekt der Erfindung liegt in der Bereitstellung eines Implantats aus einem biokorrodierbaren metallischen Werkstoff und mit einer Beschichtung oder Kavitätenfüllung bestehend aus oder enthaltend Gelatine.

Gelatine ist ein Gemisch von Polypeptiden, je nach Gewinnung mit Molmassen von ca. 13.500 bis 500.000 g/mol (bestimmt durch SDS-Gelelektrophorese oder Gelchromatographie), das durch eine mehr oder weniger weit geführte Hydrolyse von Collagen gewonnen wird. Die Aminosäure-Zusammensetzung entspricht weitgehend der des Collagens, aus dem sie gewonnen wurde, und umfasst mit Ausnahme des Tryptophans und Methionins alle essentiellen Aminosäuren; Leitaminosäure ist Hydroxyprolin. Gelatine enthält 84 bis 90 Gew.% Eiweiß und 2 bis 4 Gew.% Mineralstoffe; der Rest besteht aus Wasser. Gelatine ist geruchlos und praktisch farblos, unlöslich in Ethanol, Ethern und Ketonen, jedoch löslich in Ethylenglycol, Glycerol, Formamid und Essigsäure. Man unterscheidet zwei Herstellungsweisen: Das saure Verfahren für Gelatine des Typs A und das alkalische Verfahren für Gelatine des Typs B. Der Rohstoff für Gelatine Typ A (überwiegend Schweineschwarten) wird einem dreitätigen Aufschlussprozess unterworfen. Bei der Herstellung der Gelatine Typ B werden Rinderspalt (Mittelschicht zwischen Leder und der Unterhaut) bzw. Knochen 10 -20 Tage mit Alkali behandelt. Die Festigkeit der Gallerte wird mit einem Gelometer (texture analyzer) bestimmt und als Bloomzahl angegeben. Der isoelektrische Punkt der Gelatine liegt bei pH 7,5 bis 9,3 (Typ A) bzw. 4,7 bis 5,2 (Typ B).

Gelatine kann durch Reaktion vor allem der Amino-Gruppen mit mono- oder polyfunktionellen Reagenzien wie Acylierungsmitteln, Aldehyden, Epoxiden, HalogenVerbindungen, Cyanamid oder aktivierten ungesättigten Verbindungen chemisch modifiziert und in ihren Eigenschaften breit variiert werden. Die erhaltenen erhalten Gelatine-Derivate werden vorliegend von dem Begriff Gelatine umfasst.

In der Pharmazie und Medizin dient Gelatine zur Herstellung von weichen und harten Kapseln, von Suppositorien, als Bindemittel und Presshilfsmittel für Tabletten sowie als Stabilisator für Emulsionen und als Blutplasma-Extender. In vernetzter Form wird Gelatine zur Herstellung von sterilen, hämostatischen Schwämmen für chirurgische Zwecke, in der Kosmetik als Bestandteil von Salben, Pasten und Cremes sowie als Schutzkolloid in Shampoos, Wasch- und Reinigungsmitteln und in Gelen mit guter Hautverträglichkeit verwendet.

Bei Verwendung von Gelatine im Körper, zeigten weder Gelatine noch seine Abbauprodukte einen merklichen Effekt auf den lokalen pH-Wert. Eine Trägermatrix aus Polylactid hydrolisiert dagegen unter Ausbildung von Säurefunktionen, die für Gewebsreaktionen, wie Entzündungen, verantwortlich gemacht werden. Neben dem positiven Einfluss auf das umgebende Gewebe ist auch der Einfluss der Adukte auf die Degradation des Grundkörpers - insbesondere, wenn dieser aus Magnesium und seinen Legierungen besteht - vernachlässigbar, d. h. der Abbau des Grundkörpers wird durch die Gegenwart der Adukte nicht zusätzlich beschleunigt.

Eine Beschichtung im Sinne der Erfindung ist eine zumindest abschnittsweise Auftragung der Komponenten auf den Grundkörper des Implantats, insbesondere Stents. Vorzugsweise wird die gesamte Oberfläche des Grundkörpers des Implantats/Stents von der Beschichtung bedeckt. Alternativ kann die Gelatine in einer Kavität des Implantats/Stents bereitgestellt werden. Die Beschichtung oder Kavitätenfüllung besteht aus Gelatine oder sie enthält Gelatine. Der Gewichtsanteil der Gelatine an den die Trägermatrix bildenden Komponenten der Beschichtung oder Kavitätenfüllung beträgt mindestens 30 %, vorzugsweise mindestens 50 %, besonders bevorzugt mindestens 70 %. Die Komponenten der Beschichtung umfassen die als Trägermatrix agierenden Materialien, also Materialien die für die funktionellen Eigenschaften der Trägermatrix notwendig sind, z. B. auch Hilfsstoffe zur Verbesserung der Viskositätseigenschaften, Gelbildung und Verarbeitbarkeit. Diese Komponenten umfassen nicht die gegebenenfalls zugesetzten Wirkstoffe oder Markermaterialien.

Die erfindungsgemäß eingesetzte Gelatine ist hochgradig biokompatibel und biodegradierbar. Die Verarbeitung kann nach Standverfahren erfolgen.

Gelatine eignet sich als Trägermaterial zur Aufnahme von Wirkstoffen, insbesondere von Proteinen mit einem mittleren Molekulargewicht im Bereich von 5.000 g/mol bis 300.000 g/mol, besonders bevorzugt im Bereich von 40.000 g/mol bis 150.000 g/mol. Liegt das Molekulargewicht der Proteine unterhalb der angegebenen Grenze, so ist eine Diffusionsgeschwindigkeit der Proteine aus dem im Einsatz vorliegendem Hydrogel zu groß für die meisten lokalen therapeutischen Anwendungen. Liegt dagegen das Molekulargewicht der Proteine über dem angegebenen Höchstwert, so ist die Diffusionsgeschwindigkeit aus gleichem Grunde zu gering. Weiterhin eignet sich die Gelatine insbesondere zur Aufnahme von dODNs, Antkörpern, Flavopiridol und Amlodipin. Zur Verarbeitung wird die Gelatine durch Erwärmen, z.B. mittels Mikrowelle, verflüssigt und die Wirkstoffe werden suspendiert oder gelöst. Die Zugabe sollte vor dem Gelieren, d.h. Ausbilden eines Hydrogels erfolgen.

Alternativ oder ergänzend kann die Gelatine als Trägermatrix für Röntgenmarker oder Magnetresonanzmarker dienen. Der Röntgenmarker kann bei Implantaten aus einem biokorrodierbaren metallischen Werkstoff nicht direkt auf das Produkt aufgebracht werden, da er die Degradation des Stents durch Bildung von Lokalelementen beeinflussen würde. In der Matrix aus Gelatine ist er dagegen vom Grundkörper abgeschirmt.

Die Gelatine kann mit weiteren als Trägermatrix dienenden Materialien, zum Beispiel Polylactiden vermengt werden, um die Materialeigenschaften für die gewünschten Einsatzzwecke zu optimieren. Denkbar ist in diesem Zusammenhang auch, dass die Trägermatrix einen Schichtaufbau aufweist, z. B. eine Basisschicht aus Gelatine und eine Deckschicht aus einem weiteren Material.

Das Ansetzten der Gelatine für die Beschichtung/Befüllung von Kavitäten kann in gepufferten Lösungen erfolgen. Die Gelatine liegt vorzugsweise als 0,5 bis 20 Gew.%ige, insbesondere 2 bis 20 Gew.%ige Lösung in Wasser oder einem gepufferten wässrigen Medium vor. Diese Lösungen lassen sich besonders einfach verarbeiten. Der pH-Wert der Lösungen liegt vorzugsweise im Bereich von 5 bis 8, um eine Hydrolyse der Gelatine während der Verarbeitung zu vermeiden, was zu einer geringeren Gelfestigkeit führen würde.

Als biokorrodierbar im Sinne der Erfindung werden metallische Werkstoffe bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert. Biokorrodierbare metallische Werkstoffe im Sinne der Erfindung umfassen insbesondere Metalle und Legierungen ausgewählt aus der Gruppe der Elemente Eisen, Wolfram und Magnesium. Unter Legierung wird vorliegend ein metallisches Gefüge verstanden, deren Hauptkomponente Magnesium, Eisen oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%.

Vorzugsweise ist der biokorrodierbare Werkstoff eine Magnesiumlegierung. Insbesondere enthält die biokorrodierbare Magnesiumlegierung Yttrium und weitere Seltenerdmetalle, da sich eine derartige Legierung aufgrund ihrer physiko-chemischen Eigenschaften und hohen Biokompatibilität, insbesondere auch seiner Abbauprodukte, auszeichnet. Biodegradierbaren Magnesiumlegierungen weisen zumeist eine relativ hohe Degradationsgeschwindigkeit auf und wird eine Trägermatrix mit im Vergleich geringerer Degradationsgeschwindigkeit eingesetzt, so können unerwünschten Interaktionen zwischen den beiden Degradationsprozessen stattfinden. Diese sind jedoch mit Hinsicht auf die therapeutischen Vorgaben möglichst zu vermeiden. Es hat sich gezeigt, dass Gelatine offenbar eine höhere Degradationsgeschwindigkeit als die bisher gängigen Magnesiumlegierungen hat, so dass die angesprochenen Komplikationen vermieden werden können.

Besonders bevorzugt wird eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden.

Die metallischen Werkstoffe bzw. Magnesiumlegierungen sind so in ihrer Zusammensetzung zu wählen, dass sie biokorrodierbar sind. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl2 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37 °C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defibrillatoren.

Vorzugsweise ist das Implantat ein Stent. Stents herkömmlicher Bauart weisen eine filigrane Stützstruktur aus metallischen Streben auf, die zur Einbringung in den Körper zunächst in einem nicht-expandierten Zustand vorliegt und die am Ort der Applikation dann in einen expandierten Zustand aufgeweitet wird. Aufgrund der Verwendungsweise sind spröde Beschichtungssysteme ungeeignet; Gelatine hat dagegen besonders geeignete Materialeigenschaften, wie eine für die Zwecke hinreichende Viskosität und Flexibilität. Der Stent kann vor oder nach dem Crimpen auf einen Ballon beschichtet werden.

Ein zweiter Aspekt der Erfindung bezieht sich auf die Verwendung von Gelatine als Beschichtungsmaterial für einen Stent aus einem biokorrodierbaren metallischen Werkstoff oder als Füllung für eine Kavität in einem Stent aus einem biokorrodierbaren metallischen Werkstoff.

Stents aus der biokorrodierbaren Magnesiumlegierung WE43 (97 Gew.% Magnesium, 4 Gew.% Yttrium, 3 Gew.% Seltenerdmetalle außer Yttrium) werden wie folgt beschichtet:

Die Magnesiumoberflächen der Stents werden durch Behandlung mit einem Argonplasma aufgeraut, um eine höhere Haftigkeit des Wirkstoffs auf der Stentoberfläche zu erzielen. Alternativ oder ergänzend kann eine Oberflächenmodifizierung, z. B. durch Silanisierungen mit Methoxy- oder Ethoxysilanen oder mit Hilfe von Phosphonsäurederivaten das Haftvermögen zum metallischen Grundkörper erhöhen.

Es wird eine 10 Gew.%ige wässrige Lösung von Gelatine in Phosphat-Puffer pH 7 bei ca. 50°C angesetzt. Nach dem Abkühlen der Lösung auf ca. 30°C wird unter Rühren eine wässrige Lösung oder Dispersion eines Wirkstoffs zugegeben. Das erhaltene Gemisch wird auf den Stent gesprüht und über 24 Stunden bei Raumtemperatur getrocknet. Anschließend wird die Beschichtung durch Eintauchen in 1%ige wässrige GlutaraldehydLösung für 3 Minuten vernetzt, dann mit einer auf pH 7 gepufferten wässrigen Lösung gespült und getrocknet.

Bei der Verarbeitung methacrylierter Gelatine-Derivate wird ein Photoinitiator in die Beschichtungslösung gegeben und der Stent nach der Filmaufbringung zur Vernetzung des Films belichtet.

## Patentansprüche

1. Implantat aus einem biokorrodierbaren metallischen Werkstoff und mit einer Beschichtung oder Kavitätenfüllung bestehend aus oder enthaltend Gelatine.

2. Implantat nach Anspruch 1, bei dem der biokorrodierbare metallische Werkstoff eine Magnesiumlegierung ist.

3. Implantat nach einem der vorhergehenden Ansprüche, bei dem das Implantat ein Stent ist.

4. Verwendung von Gelatine als Beschichtungsmaterial für einen Stent aus einem biokorrodierbaren metallischen Werkstoff oder als Füllung für eine Kavität in einem Stent aus einem biokorrodierbaren metallischen Werkstoff.
